# EUROPÄISCHE PATENTSCHRIFT

(11) **EP 2 105 106 B1**
(45) Veröffentlichungstag und Bekanntmachung des Hinweises auf die Patenterteilung: **29.08.2012**
(21) Anmeldenummer: 09156211.6
(22) Anmeldetag: 25.03.2009
(51) Int. Cl.: A61B 19/00, A61B 1/12

(54) **Medizinische Reinigungsvorrichtung zum Reinigen von innenliegenden Oberflächen**
Medical cleaning device for cleaning interior surfaces
Dispositif de nettoyage médical destiné à nettoyer des surfaces internes

(30) Priorität: 26.03.2008 DE 102008016911
(43) Veröffentlichungstag der Anmeldung: 30.09.2009
(73) Patentinhaber: Karl Storz GmbH & Co. KG, 78532 Tuttlingen (DE)
(72) Erfinder: Efinger, Andreas, 78604, Rietheim (DE); Weiss, Horst, 78570, Mühlheim (DE)
(74) Vertreter: Witte, Weller & Partner

(56) Entgegenhaltungen:
- DE-A1- 10 024 133
- DE-A1-102005 048 211
- DE-U1- 29 620 011
- US-A- 4 977 900

## Beschreibung

Die Erfindung betrifft eine medizinische Reinigungsvorrichtung zum Reinigen von innenliegenden Oberflächen von Hohlschäften von medizinischen Instrumenten.

In der minimal-invasiven Medizintechnik werden medizinische Instrumente verwendet, die Hohlschäfte aufweisen.

Der Hohlschaft des medizinischen Instruments wird über eine Inzision in den Körper eingeführt und durch den Hohlschaft hindurch werden dann entsprechende Manipulationen im Körper vorgenommen.

Dabei wird die innere Oberfläche der Hohlschäfte kontaminiert und muss gereinigt werden

Ein Schritt eines Reinigungsvorganges besteht darin, eine Reinigungsflüssigkeit durch den Hohlschaft zu führen, um an der inneren Oberfläche anhaftende Kontaminationen oder Verunreinigungen abzuspülen. Unter dem Begriff Reinigungsflüssigkeit werden auch reine Spülflüssigkeiten verstanden, die bspw. auch dazu vorgesehen sein können, um Reste eines z.B. flüssigen Reinigungsmittels von der inneren Oberfläche abzuspülen.

Da die minimal-invasive Chirurgie weite Verbreitung gefunden hat, ist es üblich geworden, solche Reinigungs- und Spülvorgänge mit entsprechenden Maschinen durchzuführen. Dazu müssen die Hohlschäfte an einen Anschluss angeschlossen werden, über den der Hohlschaft mit der Reinigungs- bzw. Spülflüssigkeit versorgt wird. Da die meisten Hohlschäfte einen sehr geringen lichten Innendurchmesser aufweisen, sind die Anschlüsse mit einem relativ geringen Querschnitt versehen, bspw. mit einem Durchmesser von 3,5 mm.

Probleme bereitet die Reinigung von Hohlschäften mit relativ großem Durchmesser, somit Hohlschäfte mit einem großen Lumen. Diese haben manchmal auch von der Kreisgeometrie abweichende Querschnittsformen.

Solche medizinischen Instrumente sind bspw. Vorrichtungen zur Entnahme der Vena Saphena Magna, also der großen Beinvene, die entlang des Unter- und des Oberschenkels bis zur Leistengegend verläuft. Die Vena Saphena Magna wird häufig entnommen, um sie als Transplantat in der Koronar- und Gefäßchirurgie einzusetzen.

Der Hohlschaft dieses medizinischen Instruments weist eine Länge von etwa 45 cm und einen lichten Innendurchmesser von 5 mm auf.

Weitere großlumige Instrumente sind Operations-Tracheo-Bronchoskope, die ebenfalls Längen im Bereich von 40 cm und Innendurchmesser von bis zu 13 mm aufweisen.

Die Reinigung solcher großlumiger Hohlschäfte mit gängigen Reinigungs- bzw. Spülmaschinen mit Anschlüssen im Bereich von 3,5 mm Querschnittsdurchmesser und mit einem Spüldruck von 0,2 bar ist problematisch. Dies deswegen, da es nicht möglich ist, das Lumen vollständig mit der Reinigungs-/Spülflüssigkeit zu füllen und damit sicherzustellen, dass die gesamte innere Oberfläche ausreichend mit der Reinigungs-/Spülflüssigkeit benetzt wird.

Die Anmelderin hat Versuche dahingehend vorgenommen, in die großlumigen Hohlschäfte perforierte Hohlschäfte einzubringen, die mit zahlreichen seitlichen Austrittsöffnungen versehen sind. Die Reinigungs-/Spülflüssigkeit wird in das Innere dieses perforierten Hohlschaftes eingeführt und tritt durch die zahlreichen Öffnungen radial aus. Damit konnte erreicht werden, dass die innere Oberfläche mehr oder weniger gleichmäßig mit der Reinigungs-/Spülflüssigkeit besprüht werden kann. Im praktischen Einsatz wurde aber festgestellt, dass auch mit dieser Hilfskonstruktion ein gleichmäßiges Beaufschlagen der inneren Oberfläche eines großlumigen Hohlschaftes mit Reinigungsflüssigkeit nicht sicherzustellen ist.

Aus der US-A-4 977 900 ist eine mikrochirurgische Zange bekannt, in der eine Reinigungsvorrichtung fest und dauerhaft integriert ist. Diese Reinigungsvorrichtung dient zum Reinigen von innenliegenden Oberflächen eines Hohlschaftes des medizinischen Instruments. Ein rohrförmiger Anschluss zum Führen einer Reinigungsflüssigkeit ist von proximal in ein Gehäuse der Zange eingesetzt.

Dabei erstreckt sich ein distaler Endabschnitt des rohrförmigen Anschlusses in den inneren Hohlschaft hinein. In diesem in den inneren Hohlschaft erstreckenden Abschnitt ist ein stabförmiges Betätigungselement eingehängt, das sich mittig im Rohrschaft von dem Anschluss bis zu den beweglichen Maulteilen am distalen Ende der Schere erstreckt. Ein axiales Hin- und Herbewegen des Betätigungselementes verursacht ein Öffnen und Schließen der Maulteile der Schere.

Die Reinigungsflüssigkeit wird durch den rohrförmigen Anschluss, der sich in den Hohlschaft hinein erstreckt, eingeführt und strömt zwischen der Innenseite des Hohlschaftes und der Außenseite des mittigen stabförmigen Betätigungselements in Richtung distalem Ende. Der Austragungsquerschnitt der Mündungsöffnung des rohrförmigen Anschlusses, die ja zum großen Teil durch das Betätigungselement belegt ist, ist wesentlich geringer als das verringerte Innenlumen zwischen der Innenseite des Hohlschaftes und der Außenseite des Betätigungselementes.

Es ist Aufgabe der vorliegenden Erfindung, eine Reinigungsvorrichtung zu schaffen, mit der die innenliegende Oberfläche von Hohlschäften, insbesondere von großlumigen Hohlschäften, von medizinischen Instrumenten einwandfrei gereinigt bzw. gespült werden kann.

Erfindungsgemäß wird die Aufgabe mit einer medizinischen Reinigungsvorrichtung dadurch gelöst, dass diese einen geschlossenen Körper aufweist, der für einen Reinigungsvorgang zeitweilig in den Hohlschaft des zu reinigenden medizinischen Instrumentes einsteckbar ist, wobei ein Außendurchmesser des Körpers kleiner ist als ein lichter Innendurchmesser des Hohlschaftes, so dass ein gegenüber einem inneren Lumen des Hohlschaftes verringertes Innenlumen verbleibt, wobei der Körper einen Anschluss aufweist, über den von einem Ende ausgehend eine Reinigungsflüssigkeit von einem Ende des Hohlschaftes her in das komplette verringerte Innenlumen bei in den Hohlschaft eingesteckten Körper führbar ist, und mit einer Halterung, über die der Körper während eines Reinigungsvorganges am medizinischen Instrument zeitweilig haltbar ist.

Diese Maßnahmen zeigen nun zahlreiche Vorteile. Ein Vorteil besteht darin, dass durch Einführen des geschlossenen Körpers in den Hohlschaft des zu reinigenden Instrumentes das Lumen, das von der Reinigungs-/Spülflüssigkeit beaufschlagt werden muss, erheblich verringert ist, nämlich zu dem verringerten Innenlumen.

Es soll ja die innenliegende Oberfläche des Hohlschaftes gereinigt werden, wozu ausreichend ist, wenn über diese Oberfläche ein relativ dünner Flüssigkeitsfilm läuft, bspw. in einem Bereich von 0,3 bis 2 mm. Reinigt man einen großlumigen Hohlschaft bspw. mit einem Innendurchmesser von 15 mm und füllt diesen komplett mit durchströmendem Reinigungs-/Spülmittel, würde ohnehin die überwiegende Menge an Flüssigkeit im inneren Kern des Hohlschaftlumens axial fließen, ohne überhaupt jemals mit der zu reinigenden innenliegenden Oberfläche in Berührung zu treten. Dies führt nicht nur dazu, dass erhebliche Mengen an Reinigungs-/Spülflüssigkeit notwendig sind, sondern es ist auch später eine relativ großvolumige kontaminierte Menge an Reinigungs-/Spülflüssigkeit zu entsorgen.

Durch die zuvor erwähnte Maßnahme des Einschiebens eines geschlossenen Körpers, der das innere Lumen auf das verringerte Innenlumen reduziert, werden diese Nachteile beseitigt. Zusätzlich kann noch eine höhere Strömungsgeschwindigkeit bei gleich bleibendem Einspeisungsdruck erzielt werden, wenn dieses verringerte Innenlumen entsprechend klein ist, so dass schon durch die erhöhte Strömungsgeschwindigkeit ein zusätzlicher Reinigungseffekt dahingehend erzielt werden kann, dass anhaftende Kontaminationen, bspw. Blut, Gewebe oder sonstige Partikel, mitgerissen werden. Somit ist mit geringerer Reinigungs-/Spülflüssigkeitsmenge ein höherer Reinigungseffekt zu erzielen.

Durch Vorsehen eines Anschlusses kann die medizinische Reinigungsvorrichtung, nachdem sie in das zu reinigende Instrument eingesteckt wurde, an eine entsprechende Reinigungs-/Spülmittelleitung angeschlossen werden.

Durch Vorsehen einer Halterung, die auch als Kupplung ausgebildet sein kann, ist eine exakt ausgerichtete Halterung der Reinigungsvorrichtung am medizinischen Instrument möglich. Dadurch ist bspw. sichergestellt, dass um den in den zu reinigenden Hohlschaft eingeschobenen geschlossenen Körper ein klar definierter Raum verbleibt, durch den die Reinigungs-/Spülflüssigkeit strömen kann, so dass sichergestellt ist, dass die gesamte innenliegende Oberfläche des Hohlschaftes mit der Flüssigkeit beaufschlagt wird. Die Halterung oder Kupplung kann z.B. Schrauben, Stifte oder eine Rastverbindung umfassen. Dies erleichtert die Handhabung der Reinigungsvorrichtung bzw. den Zusammenbau aus Reinigungsvorrichtung und Schaft.

Es können somit auch großlumige Hohlschäfte in gängigen Reinigungs-/Spülmaschinen bearbeitet werden, indem nämlich durch das zusätzliche Einführen der erfindungsgemäßen medizinischen Reinigungsvorrichtung das verringerte freie Innenlumen geschaffen wird, dennoch die gesamte innenliegende Oberfläche freiliegend für den Reinigungs- bzw. Spülvorgang ist.

In einer weiteren Ausgestaltung der Erfindung ist die Geometrie des Körpers so gewählt, dass der Querschnitt des verringerten Innenlumens kleiner oder gleich dem Querschnitt des Anschlusses ist.

Unter diesen Bedingungen kann sichergestellt werden, dass die über den Anschluss bzw. dessen Querschnitt zugeführte Reinigungs-/Spülflüssigkeit das komplette verringerte Innenlumen belegen kann, so dass keine Hohl- oder Luftblasenräume entstehen können, die nicht von der Flüssigkeit beaufschlagt werden. Durch die entsprechende Wahl der Geometrie können auch die unterschiedlichsten großlumigen Hohlschäfte in gängigen, d.h. bereits vorhandenen, Maschinen mit standardisierten Anschlüssen, z.B. mit 3,5 mm Querschnitt, gereinigt und gespült werden.

In einer weiteren Ausgestaltung der Erfindung weist der Körper an seiner Außenseite strömungsbeeinflussende Elemente auf.

Diese Maßnahme hat den Vorteil, dass durch diese Elemente die Strömung bzw. das Strömungsprofil der Flüssigkeit durch das verringerte Innenlumen beeinflusst werden kann. Diese strömungsbeeinflussenden Elemente können bspw. der Flüssigkeit eine Art schraubenlinienförmige Bewegung auferlegen, so dass der Flüssigkeitsstrom in einer solchen Bewegungscharakteristik durch das verringerte Innenlumen strömt, wodurch bestimmte Reinigungs- und Spüleffekte erzielt werden können. Wenn man nur flüssige Kontaminationen abspülen möchte, kann es ausreichend sein, eine laminare Strömung zu bewerkstelligen. Wenn an der zu reinigenden innenliegenden Oberfläche des Hohlschaftes klebrige Partikel anhaften, bspw. Gewebestücke oder dergleichen, kann es auch wünschenswert sein, durch die strömungsbeeinflussenden Elemente ganz gezielt eine turbulente Strömung zu erzeugen, um zusätzliche Reinigungswirkung bzw. Ablöseffekte durch die turbulenten Wirbel zu erzielen. Es ist also möglich, ganz individuell auf den Einsatzbereich des medizinischen Instruments abzuzielen.

In einer Ausgestaltung der Erfindung sind die strömungsbeeinflussenden Elemente insbesondere ausgewählt aus Vorsprüngen, vorzugsweise finnenartige oder ringartige Vorsprünge, schraubenlinienförmige Vorsprünge, Vertiefungen, vorzugsweise ringartige oder schraubenlinienförmige Vertiefungen, Aufrauungen an der Außenseite des in den Hohlschaft einzuschiebenden Körpers.

Durch Vorsehen dieser unterschiedlichen strömungsbeeinflussenden Elemente kann in Abhängigkeit von dem Einsatz des Instrumentes, von der Geometrie des Instrumentes und von den allfälligen Kontaminationen ganz gezielt eine bestimmte Strömungscharakteristik erzielt werden, die jeweils für den zu erzielenden Reinigungs- und Spüleffekt am günstigsten ist.

In einer weiteren Ausgestaltung der Erfindung ist der Körper stabförmig.

Diese Maßnahme hat den Vorteil, dass der Körper sehr einfach ausgebildet und auch einfach maßhaltig herzustellen ist und einfach handzuhaben ist. Die hohe Maßhaltigkeit erlaubt bspw. ein stark verringertes Innenlumen, bspw. von einem Querschnittsradius von 15 mm auf 2 mm zu verringern, wobei durch die vorgesehene Halterung der Körper dann exakt in dieser Ausrichtung gehalten werden kann. Auch das Einschieben eines stabförmigen Körpers in einen Hohlschaft ist ein einfach durchzuführender Vorgang.

In einer weiteren Ausgestaltung der Erfindung ist die Länge des Körpers durch aneinanderfügen von Körperabschnitten veränderbar.

Diese Maßnahme hat den Vorteil, dass die Länge des Körpers variiert werden kann, um diesen an unterschiedliche Längen von Hohlschäften anzupassen. Es ist gängig, dass im medizinischen Bereich die Länge von Hohlschäften in Abhängigkeit von der Körpergröße der zu behandelnden Patienten gewählt wird, das heißt, es werden unterschiedliche Längen bei Kindern, Jugendlichen oder Erwachsenen eingesetzt. Es ist dann ausreichend, einen Grundkörper mit der Halterung und dem Anschluss an einem Ende zu versehen, und den Körper durch Anfügen von weiteren Körperabschnitten am gegenüberliegenden Ende zu verlängern oder entsprechend zu verkürzen. Hier eröffnet sich auch die Möglichkeit, nun Körperabschnitte mit unterschiedlichen Oberflächengestaltungen aneinander zu koppeln, so dass ein Wechsel von laminaren, turbulenten oder sonst wie gerichteten Strömungen nach und nach erzielt werden können. Dies zeigt die hohe Flexibilität der erfindungsgemäßen Vorrichtung gegenüber unterschiedlichen Reinigungs-/Spülanforderungen.

In einer weiteren Ausgestaltung der Erfindung entspricht die Querschnittsgeometrie des Körpers der Querschnittsgeometrie des Hohlschaftes.

Die überwiegende Zahl der Hohlschäfte werden einen kreisförmigen Querschnitt aufweisen, so dass dann auch der eingeschobene stabförmige Körper einen kreisförmigen Querschnitt aufweist. Dadurch entsteht dann bei eingeschobenen stabförmigem Körper ein ringförmiges verringertes Innenlumen mit einer gleich bleibenden Geometrie bezüglich der Dicke. Ist der Hohlschaft nicht kreisförmig, sondern oval oder sonst wie geformt, kann dann entsprechend die Geometrie des Querschnitts des Körpers ausgeformt sein, um durchgehend ein verringertes Innenlumen mit gleich bleibender Dicke zu schaffen.

In einer weiteren Ausgestaltung der Erfindung sind bei unregelmäßig geformten Hohlschäften mit dadurch vorhandenen, schwierig zu reinigenden Innenflächenbereichen am Körper der Reinigungsvorrichtung zumindest im Bereich dieses Innenflächenbereiches strömungsbeeinflussende Elemente vorhanden, die eine turbulente Strömung der Reinigungsflüssigkeit verursachen.

Diese Maßnahme hat den erheblichen Vorteil, dass bei unregelmäßig geformten Hohlschäften und damit möglicherweise verbundenen, schwierig zu reinigenden Innenflächenbereichen gezielte Verwirbelungen erzielt werden können, um auch in diesem kritischen Bereich anhaftende Kontaminationen sicher ablösen zu können. Hier zeigt sich erneut die hohe Anpassungsfähigkeit und auch Individualität der Vorrichtung an problematisch zu reinigende Hohlschäfte.

In einer weiteren Ausgestaltung der Erfindung besteht der Körper aus einem biegsamen Material, so dass er auch in einen gekrümmten Hohlschaft einsteckbar ist.

Diese Maßnahme hat den Vorteil, dass auch gekrümmte großlumige Hohlschäfte unter Einsatz der erfindungsgemäßen Vorrichtung mit einem hervorragenden Ergebnis gereinigt und gespült werden können. Vorteilhaft ist diese Ausgestaltung auch in Zusammenhang mit abgewinkelten Schäften und solchen, deren Querschnitt sich axial verändert.

In einer weiteren Ausgestaltung der Erfindung weist der Körper aus biegsamem Material an seiner Außenseite Leitelemente auf, die ein Einführen und Positionieren des Körpers im gekrümmten Hohlschaft erleichtern.

Diese Maßnahme hat den Vorteil, dass die Leitelemente nicht nur das Hineinschieben und das Herausziehen des Körpers aus biegsamem Material in den/aus dem zu reinigenden Hohlschaft erleichtern, sondern gleichzeitig auch dafür sorgen, dass der Körper gleichmäßig von der zu reinigenden Innenfläche beabstandet werden kann, so dass wieder durchweg ein relativ gleichmäßiges, verringertes Innenlumen geschaffen wird. Diese Leitelemente sorgen auch bei geradlinigen Schäften für eine exakte Positionierung.

In einer weiteren Ausgestaltung der Erfindung ist im Bereich des Anschlusses am Körper zumindest eine Öffnung vorhanden, über die die Reinigungs- bzw. Spülflüssigkeit in das verringerte Innenlumen strömt.

Diese Maßnahme hat den Vorteil, dass die über den Anschluss zugeführte Flüssigkeit definiert über diese Öffnung in das verringerte Innenlumen einströmen kann, dort gleichmäßig verteilt werden kann und den Hohlschaft dann gleichmäßig verteilt von der einen Seite, an der der Anschluss angeordnet ist, bis zur gegenüberliegenden Austrittsseite für die Flüssigkeit gleichmäßig durchströmt.

Es versteht sich, dass die vorstehend genannten und die nachstehend noch zu erläuternden Merkmale nicht nur in den jeweils angegebenen Kombinationen, sondern auch in anderen Kombinationen oder in Alleinstellung einsetzbar sind, ohne den Rahmen der vorliegenden Erfindung zu verlassen.

Ausführungsbeispiele der Erfindung werden nachstehend unter Bezugnahme auf die beiliegenden Zeichnungen näher beschrieben und erläutert. Es zeigen:
- Fig. 1: eine Draufsicht eines ersten Ausführungsbeispiels einer medizinischen Reinigungsvorrichtung,
- Fig. 2: einen um 90° um die Längsachse verdrehten Längsschnitt der Vorrichtung von Fig. 1,

- Fig. 3: einen Längsschnitt eines medizinischen Instrumentes, nämlich eine Vorrichtung zur Entnahme der Vena Saphena Magna, deren Hohlschaft gereinigt werden soll,
- Fig. 4: eine der Fig. 3 vergleichbare Schnittdarstellung mit der Reinigungsvorrichtung von Fig. 2 in den Hohlschaft eingeschoben,
- Fig. 5: eine stark vergrößerte Schnittdarstellung des in Fig. 4 mit einem Kreis umgrenzten Bereichs,
- Fig. 6: einen Schnitt längs der Linie VI-VI in Fig. 5,
- Fig. 7: eine perspektivische Ansicht des medizinischen Instruments von Fig. 3, bei der gerade die Vorrichtung von Fig. 1 in den Hohlschaft eingeschoben wird,
- Fig. 8: eine der Darstellung von Fig. 7 vergleichbare ausschnittsweise Darstellung mit vollständig in den Hohlschaft eingeschobener medizinischer Reinigungsvorrichtung,
- Fig. 9: eine der Darstellung von Fig. 1 vergleichbare Darstellung eines zweiten Ausführungsbeispiels mit Vertiefungen am stabförmigen Körper,
- Fig. 10: eine der Darstellung von Fig. 4 vergleichbare Darstellung, bei der die medizinische Reinigungsvorrichtung von Fig. 9 in den Hohlschaft des Instrumentes von Fig. 3 eingeschoben ist,
- Fig. 11: eine der Darstellung von Fig. 5 entsprechend vergrößerte Darstellung des in Fig. 10 mit einem Kreis umgrenzten Bereiches,

- Fig. 12: eine den Darstellungen von Fig. 5 und 11 vergleichbare Darstellung eines dritten Ausführungsbeispieles, bei dem die Außenseite des stabförmigen Körpers mit Vorsprüngen versehen ist,
- Fig. 13: eine der Darstellung von Fig. 12 vergleichbare Darstellung eines vierten Ausführungsbeispiels,
- Fig. 14: ein fünftes Ausführungsbeispiel einer medizinischen Reinigungsvorrichtung mit einem stabförmigen Körper aus biegsamem Material, der in einen gekrümmten Hohlschaft eingeschoben ist,
- Fig. 15: eine stark vergrößerte abschnittsweise Darstellung von Fig. 14, bei der ersichtlich ist, dass an der Außenseite Vorsprünge vorhanden sind,
- Fig. 16: einen Querschnitt durch ein Instrument mit nichtrundem Hohlschaft und einem entsprechend geformten, darin eingeschobenen stabförmigen Körper, und
- Fig. 17: eine der Fig. 16 vergleichbare Darstellung, bei der der eingeschobene stabförmige Körper der Reinigungsvorrichtung zusätzlich Leitelemente aufweist.

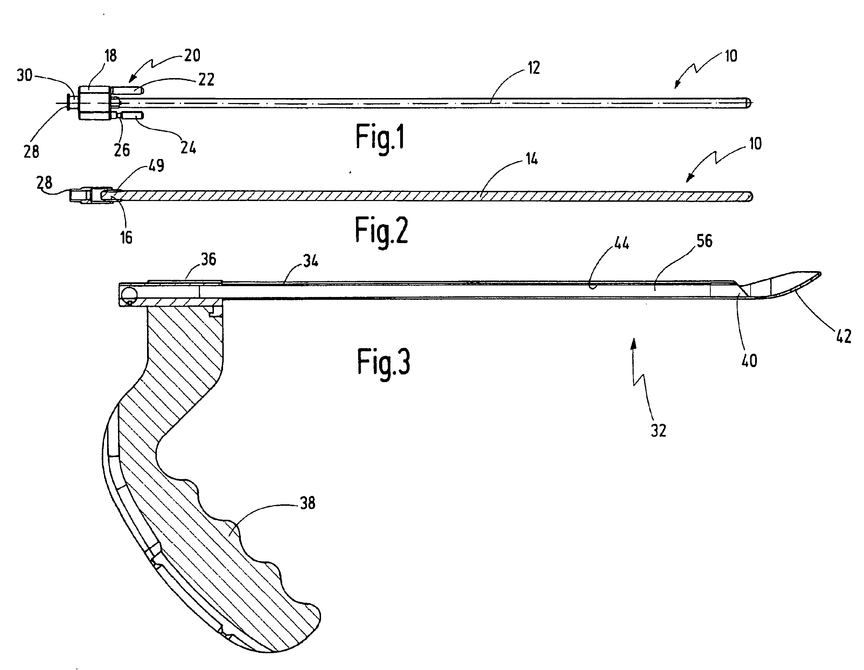

Ein in den Figuren 1 bis 8 dargestelltes erstes Ausführungsbeispiel einer erfindungsgemäßen medizinischen Reinigungsvorrichtung ist in ihrer Gesamtheit mit der Bezugsziffer 10 bezeichnet.

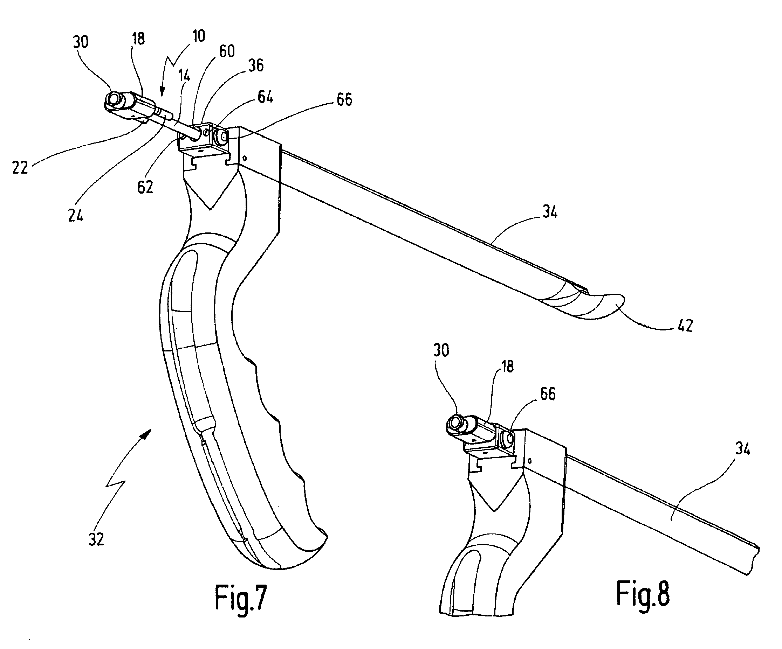

Wie insbesondere aus den Figuren 1 und 2 ersichtlich, weist die Vorrichtung 10 einen langerstreckten Körper 12 in Form eines massiven Stabes 14, hier aus Stahl, auf. An einem Ende 16 ist der Stab mit einem Gehäuse 18 verbunden. Das Gehäuse 18 weist eine Halterung 20 auf, deren Funktionsweise später in Zusammenhang mit den Figuren 7 und 8 beschrieben werden soll.

Die Halterung 20 weist zwei von dem Gehäuse 18 vorspringende Stifte 22 und 24 auf, die sich beidseits des Stabes 14 und parallel und im Abstand zu diesem erstrecken. Der Stift 24 ist mit einer umlaufenden Ringnut 26 versehen.

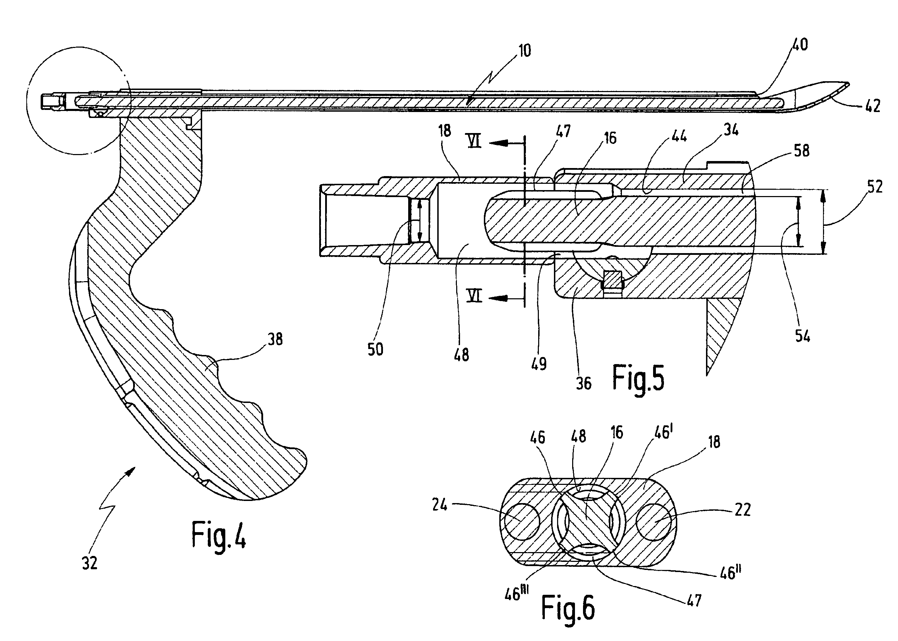

Am proximalen Ende des Gehäuses 18 ist ein Anschluss 28 vorgesehen, der als ein sog. LUER-Anschluss 30 ausgebildet ist. Ein LUER-Anschluss 30 ist ein standardisierter Anschluss im medizinischen Bereich, an den durch eine 90°-Drehung ein entsprechend geformter Anschluss bspw. einer Flüssigkeitsleitung angeschlossen werden kann. Wie aus der Schnittdarstellung von Fig. 2 zu erkennen, ist das Gehäuse 18 innen hohl und mit dem Anschluss 28 verbunden. Der Stab 14 reicht in das Gehäuse 18 hinein, erstreckt sich also in die innere Bohrung 48, wie das aus Fig. 5 an der linken Seite ersichtlich ist, hinein. Aus Fig. 6 ist ersichtlich, dass dieses hintere Ende 16 des Stabes 14 vier Stege 46, 46', 46" und 46"' aufweist, über die sich dieses Ende an der Innenseite des Gehäuses 18 abstützt und damit verbunden ist. Zwischen den Stegen 46, 46', 46" und 46"' sind somit Öffnungen, hier ist nur die Öffnung 47 angedeutet, durch die eine Flüssigkeit, die dem Gehäuse 18 über den Anschluss 28 zugeführt wurde, durchtreten kann.

Die erfindungsgemäße Vorrichtung 10 dient dazu, in Zusammenhang mit einem medizinischen Instrument 32 verwendet zu werden, wie es in Fig. 3 im Längsschnitt dargestellt ist. Das medizinische Instrument 32 ist eine Vorrichtung zur Entnahme der Vena Saphena Magna aus dem Bein eines erwachsenen Menschen. Das medizinische Instrument 32 weist einen langerstreckten Hohlschaft 34 auf, dessen Länge etwa 45 cm und dessen lichter Innendurchmesser etwa 5 mm beträgt. Am proximalen Ende 36 ist der Hohlschaft mit einem Griff 38 verbunden, am distalen Ende 40 mit einer Spatelspitze 42 versehen.

Der Hohlschaft 34 weist eine innere Oberfläche 44 auf, die nach einem Einsatz, also nach Entnahme der Beinvene, gereinigt und gespült werden soll.

Dazu wird die Vorrichtung 10 in das proximale offene Ende 36 des Hohlschaftes 34 eingeschoben.

In Fig. 7 ist eine Situation dargestellt, bei der der Stab 14 der Vorrichtung 10 schon zum großen Teil durch eine Öffnung 60 am proximalen Ende 36 des Hohlschaftes 34 eingeschoben worden ist. An diesem proximalen Ende sind beidseits der Öffnung 60 noch zwei Öffnungen 62 und 64 vorgesehen, die dazu dienen, dass in diese die vorspringenden Stifte 22 und 24 der Halterung 20 eingeschoben werden können. An der Seite, an der der Stift 24 eingeschoben wird, der mit der Ringnut versehen ist, ist ein quer vorstehender, federbelasteter Arretierstift 66 vorgesehen.

Ist die Vorrichtung 10 vollständig in den Hohlschaft 34 eingeschoben, sind die beiden Stifte 22 und 24 in die entsprechenden Öffnungen 62 und 64 eingetreten und der Arretierstift 66 ist in die Ringnut 26 des Stiftes 24 eingerastet. Diese Endposition ist in Fig. 8 dargestellt.

Aus der Schnittdarstellung von Fig. 5 ist ersichtlich, dass das distale Ende des Gehäuses 18 dabei bündig und dichtend abschließend an dem proximalen Ende 36 des medizinischen Instrumentes 32 anliegt.

Aus der Schnittdarstellung von Fig. 5 ist auch zu erkennen, dass der Außendurchmesser 54 des Stabes 14 geringer ist als der lichte Innendurchmesser 52 des Hohlschaftes 34. Somit wurde das innere Lumen 56 des Hohlschaftes 34 (siehe Fig. 3) auf ein verringertes Innenlumen 58 reduziert.

Die Bemaßung ist dabei so gewählt, dass der Querschnitt des Innenlumens 58 maximal gleich oder kleiner dem Querschnitt 50 des LUER-Anschlusses 30 ist.

Dadurch ist sichergestellt, dass eine durch den LUER-Anschluss 30 zugeführte Flüssigkeit komplett das Innenlumen 58 des nun verbleibenden Ringraumes ausfüllt. Die zur reinigende innere Oberfläche 44 des Hohlschaftes 34 wird komplett mit der Flüssigkeit beaufschlagt und allfällige Kontaminationen werden abgespült oder mitgerissen und bis zum distalen Ende 40 des medizinischen Instrumentes 32 geführt und dort im Bereich der Spatelspitze 42 abgeführt. Durch die Halterung 20 ist sichergestellt, dass die Vorrichtung 10 unverlierbar und fest am medizinischen Instrument 32 für den Reinigungs- und Spülvorgang gehalten ist, und zwar in einer bestimmten geometrischen Außenrichtung, die zu dem geometrisch exakt gleichmäßigen ringförmigen verringerten Innenlumen 58 führt.

In dem in Fig. 8 gezeigten Zustand kann der Zusammenbau bspw. in eine Reinigungs- und Spülmaschine gegeben werden. An den LUER-Anschluss 30 wird eine Spül- bzw. Reinigungsflüssigkeitsleitung angeschlossen und dann kann der weitere Vorgang automatisiert durchgeführt werden.

Nach Beendigung des Vorganges wird der Arretierstift 66 seitlich bewegt und die Vorrichtung 10 kann vom Hohlschaft 34 wieder abgezogen werden.

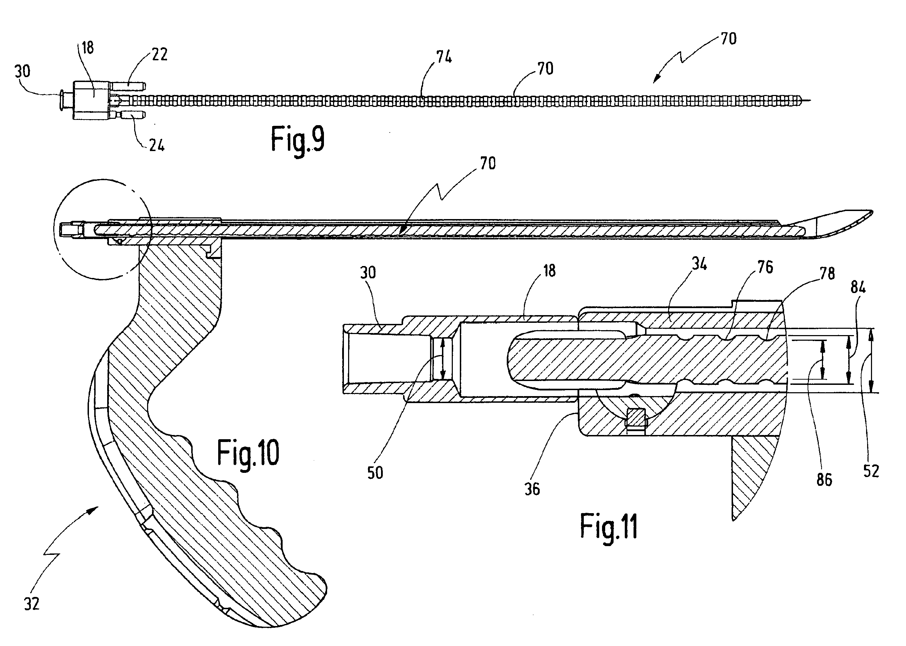

In den Figuren 9 bis 11 ist ein zweites Ausführungsbeispiel einer erfindungsgemäßen Vorrichtung 70 dargestellt, die sich von der zuvor beschriebenen Vorrichtung 10 einzig dadurch unterscheidet, dass der Stab 74 Vertiefungen 76 in Form von ringförmigen Kerben 78 aufweist. Ansonsten ist die Vorrichtung 70 baugleich zu der Vorrichtung 10, daher werden auch für diese Bauteile gleiche Bezugszeichen verwendet. Das heißt, auch hier ist an einem Ende ein Gehäuse 18 vorhanden, das mit dem LUER-Anschluss 30 versehen ist. Die Halterung besteht wieder aus den beiden vorstehenden Stiften 22 und 24.

In Fig. 10 und 11 ist dargestellt, wie die Vorrichtung 70 in das schon zuvor beschriebene medizinische Instrument 32, nämlich die Vorrichtung zur Entnahme eine Vena Saphena Magna, eingeschoben ist.

Aus der Schnittdarstellung von Fig. 11 ist zu erkennen, dass die konstruktive Ausgestaltung des Gehäuses 18 und die Anlage an das proximale Ende 36 des medizinischen Instrumentes gleichermaßen ausgestaltet sind, lediglich der Stab 74 weist die beschriebenen Kerben 78 auf. Auch hier ist wieder der Außendurchmesser 84 des Stabes 74 kleiner als der lichte Innendurchmesser 52 des Hohlschaftes 34, so dass auch hier wieder ein hier nicht näher bezeichnetes reduziertes Innenlumen entsteht. Dieses Innenlumen ist nun dahingehend etwas vergrößert, dass die Flüssigkeit auch den Raum der ringförmigen Kerben 78 ausfüllen muss, da ja der Bodendurchmesser 86 noch etwas geringer ist als der Außendurchmesser 34.

Durch das Vorsehen dieser Kerben 78 können turbulente Strömungen, also entsprechende Wirbel, erzeugt werden, um den Reinigungs- bzw. Spüleffekt zu erhöhen.

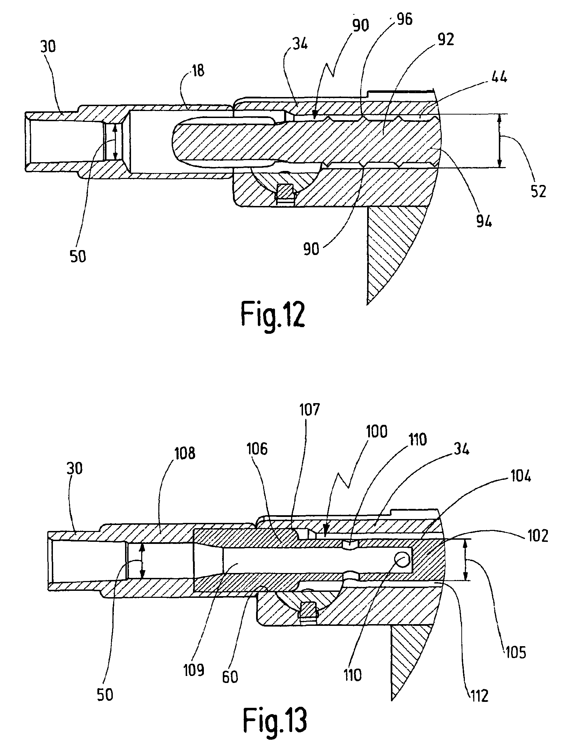

Bei dem in Fig. 12 gezeigten dritten Ausführungsbeispiel einer erfindungsgemäßen Vorrichtung 90 ist wieder ein Körper 92 in Form eines Stabes 94 vorhanden, an dessen Außenseite Vorsprünge 96 vorstehen.

Ansonsten ist die Vorrichtung 90 gleichermaßen wie die zuvor beschriebenen Vorrichtungen 10 bzw. 70 ausgebildet, das heißt, es ist wieder ein Gehäuse 18 vorhanden, das in einem LUER-Anschluss 30 mündet. Der Außendurchmesser des Stabes 94 ist dann wieder etwas geringer als der lichte Innendurchmesser 52 des Hohlschaftes 34 des medizinischen Instrumentes. Sind die Vorsprünge 96 als ringförmig umlaufende Vorsprünge ausgebildet, steht die Spitze des Vorsprunges 96 in einem gewissen Abstand zur inneren Oberfläche 44, so dass ein Flüssigkeitsdurchtritt erfolgen kann.

Die Vorsprünge können auch als eine Art schraubenlinienförmiges Außengewinde ausgebildet sein. Im Bereich der Vorsprünge 96 entstehen erhebliche Verwirbelungen, die ein zusätzliches Abspülen von insbesondere klebrigen oder schleimigen Kontaminationen fördert.

In Fig. 13 ist ein viertes Ausführungsbeispiel einer erfindungsgemäßen Vorrichtung 100 dargestellt. Auch hier ist ein langerstreckter, geschlossener, massiver Körper 102 vorhanden, der die Form eines Stabes 104 aufweist, der an seiner Außenseite glatt ist und dessen Durchmesser 105 wieder etwas geringer ist als der lichte Innendurchmesser des Hohlschaftes 34 des Instrumentes.

Im Unterschied zu den zuvor gezeigten Ausführungsbeispielen ist die Halterung 106 hier so ausgebildet, dass sie als eine Art Stopfen 107 ausgebildet ist, der direkt in ein Ende des entsprechenden Hohlschaftes 34 eingesteckt werden kann. Dazu ist das Material in diesem Bereich etwa weichelastisch ausgebildet. Auf das äußere Ende des Stopfens 107 ist dann der Anschluss 108 aufgesetzt. Der Stopfen 107 sorgt für einen flüssigkeitsdichten Abschluss.

Daher ist eine mittige zentrale Bohrung 109 vorhanden, die mit Öffnungen 110 in Verbindung steht.

Die Lage der Öffnungen 110 ist so, dass diese in das reduzierte Innenlumen 112 münden.

Ist also die Vorrichtung 100 in ein medizinisches Instrument 32 eingeschoben, wird eine Flüssigkeit über den Anschluss 108 die innere Bohrung 109 im Stopfen und die seitlichen Öffnungen 110 in das reduzierte Innenlumen 112 geführt.

Dazu ist auch hier vorgesehen, dass der Außendurchmesser 105 des Stabes 104 wieder geringer ist als der lichte Innendurchmesser des zu reinigenden Hohlschaftes 34.

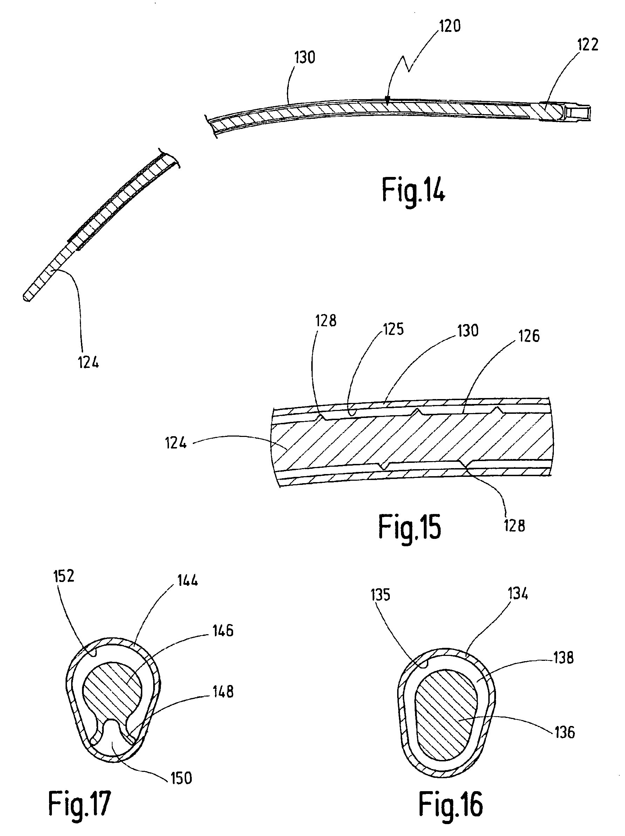

Bei einem weiteren in Figuren 14 und 15 dargestellten fünften Beispiel einer erfindungsgemäßen Vorrichtung 120 weist diese einen Körper 122 aus flexiblem bzw. biegsamem Material auf.

Auch hier ist der Körper 122 als längserstreckender Stab 124 ausgebildet, an dessen Außenseite 126 Leitelemente 128 in Form von Vorsprüngen 129 vorstehen. Durch diese Ausgestaltung kann die Vorrichtung 120 einfach in einen gekrümmten Hohlschaft 130 eingeschoben werden. Die Leitelemente 128 sorgen dafür, dass auch in dem gekrümmten Hohlschaft 130 der Körper 120 gleichmäßig beabstandet von der zu reinigenden inneren Oberfläche 125 des Hohlschaftes 130 liegt.

Diese Leitelemente 128 haben zusätzlich noch den Effekt, wie zuvor mit dem Ausführungsbeispiel in Zusammenhang mit Fig. 12 beschrieben, das heißt, dass entsprechende Turbulenzen im Bereich der Vorsprünge erzielt werden können. Allerdings liegen hier die Vorsprünge zu Stützzwecken an der Innenseite der Oberfläche 125 punktuell an.

In Fig. 16 ist ein sechstes Ausführungsbeispiel gezeigt, bei dem ein Hohlschaft 134 dargestellt ist, der nicht kreisförmig ist, sondern als Oval mit einer kleineren Rundungsseite. Hier ist der Körper 136 der entsprechenden erfindungsgemäßen Vorrichtung geometrisch ähnlich ausgebildet, aber in seinen Ausmaßen etwas geringer, so dass dann wieder das verringerte Innenlumen 138 entsteht, wenn der Körper 136 in den Hohlschaft 134 des zu reinigenden Instrumentes eingeschoben ist. Auch hier kann dann die innere Oberfläche 135 mit gutem Ergebnis gereinigt werden.

Bei dem in Fig. 17 dargestellten siebten Ausführungsbeispiel ist der Hohlschaft 144 ähnlich geformt, wie der von Ausführungsbeispiel von Fig. 16, der Körper 146 der in den Hohlschaft 144 eingeschobenen erfindungsgemäßen Vorrichtung, ist aber stabförmig mit etwa kreisförmigem Querschnitt. Von der Außenseite stehen Leitelemente 148 vor, die in dem möglicherweise kritischen, das heißt schwierig zu reinigenden Innenflächenbereich 150, für entsprechende Turbulenzen bzw. Verwirbelungen sorgen. Somit ist auch hier sichergestellt, dass die gesamte innere Oberfläche 152 mit hervorragendem Ergebnis gereinigt werden kann.

## Patentansprüche

1. Medizinische Reinigungsvorrichtung zum Reinigen von innenliegenden Oberflächen (44, 125, 135, 152) von Hohlschäften (34, 130, 134, 144) von medizinischen Instrumenten (32), mit einem geschlossenen Körper (12, 92, 102, 122, 136, 146), der für einen Reinigungsvorgang zeitweilig in den Hohlschaft (34, 130, 134, 144) des zu reinigenden medizinischen Instrumentes (32) einsteckbar ist, wobei ein Außendurchmesser (54, 84) des Körpers kleiner ist als ein lichter Innendurchmesser (52) des Hohlschaftes (34, 130, 134, 144), so dass ein gegenüber einem inneren Lumen des Hohlschaftes (34, 130, 134, 144) verringertes Innenlumen (58, 112, 138) verbleibt, wobei der Körper (12, 92, 102, 122, 136, 146) einen Anschluss (28) aufweist, über den von einem Ende (16) ausgehend eine Reinigungsflüssigkeit von einem Ende (36) des Hohlschaftes (34, 130, 134, 144) her in das komplette verringerte Innenlumen (58, 112, 138) bei in den Hohlschaft (34, 130, 134, 144) eingestecktem Körper (12, 92, 102, 122, 136, 146) führbar ist, und mit einer Halterung (20, 106), über die der Körper (12, 92, 102, 122, 136, 146) während eines Reinigungsvorganges am medizinischen Instrument (32) zeitweilig haltbar ist.

2. Medizinische Reinigungsvorrichtung nach Anspruch 1, **dadurch gekennzeichnet, dass** die Geometrie des Körpers (12, 92, 102, 122, 136, 146) so gewählt ist, dass ein Querschnitt des verringerten Innenlumens (58, 112, 138) kleiner oder gleich dem Querschnitt (50) des Anschlusses (28) ist.

3. Medizinische Reinigungsvorrichtung nach Anspruch 1 oder 2, **dadurch gekennzeichnet, dass** der Körper an seiner Außenseite strömungsbeeinflussende Elemente (76, 78, 96, 128) aufweist.

4. Medizinische Reinigungsvorrichtung nach Anspruch 3, **dadurch gekennzeichnet, dass** die strömungsbeeinflussenden Elemente insbesondere ausgewählt sind aus Vorsprüngen (96), vorzugsweise finnenartige oder ringartige Vorsprünge, schraubenlinienförmige Vorsprünge, Vertiefungen (76), insbesondere ringartige oder schraubenlinienförmige Vertiefungen, Aufrauungen.

5. Medizinische Reinigungsvorrichtung nach einem der Ansprüche 1 bis 4, **dadurch gekennzeichnet, dass** der Körper (12, 92, 102, 122, 136, 146) stabförmig ist.

6. Medizinische Reinigungsvorrichtung nach einem der Ansprüche 1 bis 5, **dadurch gekennzeichnet, dass** die Länge des Körpers durch aneinanderfügen von Körperabschnitten veränderbar ist.

7. Medizinische Reinigungsvorrichtung nach einem der Ansprüche 1 bis 6, **dadurch gekennzeichnet, dass** die Querschnittsgeometrie des Körpers (12, 92, 102, 122, 136) der Querschnittsgeometrie des Hohlschaftes (34, 130, 134) entspricht.

8. Medizinische Reinigungsvorrichtung nach einem der Ansprüche 1 bis 6, **dadurch gekennzeichnet, dass** bei unregelmäßig geformten Hohlschäften (144) mit dadurch vorhandenen, schwierig zu reinigenden Innenoberflächenbereichen (150) am Körper (146) der Reinigungsvorrichtung zumindest im Bereich dieses Innenflächenbereiches (150) strömungsbeeinflussende Elemente (128) vorhanden sind, die eine turbulente Strömung der Reinigungsflüssigkeit verursachen.

9. Medizinische Reinigungsvorrichtung nach einem der Ansprüche 1 bis 8, **dadurch gekennzeichnet, dass** der Körper (122) aus einem biegsamen Material besteht, so dass er auch in gekrümmte Hohlschäfte (130) einsteckbar ist.

10. Medizinische Reinigungsvorrichtung nach Anspruch 9, **dadurch gekennzeichnet, dass** der Körper (122) aus biegsamem Material an seiner Außenseite (126) Leitelemente (128) aufweist, die ein Einführen und Positionieren des Körpers (122) in gekrümmte Hohlschäfte (130) erleichtern.

11. Medizinische Reinigungsvorrichtung nach einem der Ansprüche 1 bis 10, **dadurch gekennzeichnet, dass** im Bereich des Anschlusses (28) am Körper zumindest eine Öffnung (110) vorhanden ist, über die die Reinigungsflüssigkeit in das verringerte Innenlumen (112) strömt.

12. Medizinische Reinigungsvorrichtung nach einem der Ansprüche 1 bis 11, **dadurch gekennzeichnet, dass** die Halterung als Kupplung ausgebildet ist.

## Claims

1. Medical cleaning device for cleaning interior surfaces (44, 125, 135, 152) of hollow shafts (34, 130, 134, 144) of medical instruments (32), with a closed body (12, 92, 102, 122, 136, 146) which can be inserted temporarily for a cleaning process into the hollow shaft (34, 130, 134, 144) of the medical instrument (32) to be cleaned, an outer diameter (54, 84) of the body being smaller than a clear inner diameter (52) of the hollow shaft (34, 130, 134, 144) such that an inner lumen (58, 112, 138) remains which is reduced compared to an inner lumen of a hollow shaft (34, 130, 134, 144), the body (12, 92, 102, 122, 136, 146) having a connection (28) by means of which, starting from one end (16) a cleaning liquid can be guided into the reduced inner lumen (58, 112, 138) when the body (12, 92, 102, 122, 136, 146) is inserted in the hollow shaft (34, 130, 134, 144), and with a support (20, 106) by means of which the body (12, 92, 102, 122, 136, 146) can be supported on the medical instrument (32).

2. Medical instrument of claim 1, **characterized in that** the geometry of the body (12, 92, 102, 122, 136, 146) is selected such that a cross section of the reduced inner lumen (58, 112, 138) is smaller than or equal to the cross section (50) of the connection (28).

3. Medical cleaning device of claims 1 or 2, **characterized in that** the body has flow-influencing elements (76, 78, 96, 128) on its outside.

4. Medical cleaning device of claim 3, **characterized in that** the flow-influencing elements are in particular selected from projections (96), preferably fin-like or annular projections, helical projections, recesses (76), in particular annular or helical recesses, roughenings.

5. Medical cleaning device of any one of claims 1 through 4, **characterized in that** the body (12, 92, 102, 122, 136, 146) is rod-shaped.

6. Medical cleaning device of any one of claims 1 through 5, **characterized in that** the length of the body can be changed by joining body sections.

7. Medical cleaning device of any one of claims 1 through 6, **characterized in that** the cross-sectional geometry of the body (12, 92, 102, 122, 136) corresponds to the cross-sectional geometry of the hollow shaft (34, 130, 134).

8. Medical cleaning device of any one of claims 1 through 6, **characterized in that** the cleaning device has flow-influencing elements (128) causing a turbulent flow of the cleaning liquid in the case of irregularly shaped hollow shafts (144) which, as a result of being irregular, have inner surface regions (150) of the body (146) of the cleaning device which are hard to clean, said elements being present at least in the region of this inner surface region (150).

9. Medical cleaning device of any one of claims 1 through 8, **characterized in that** the body (122) is composed of a flexible material such that it can also be inserted into curved hollow shafts (130).

10. Medical instrument of claim 9, **characterized in that** the body (122) composed of a flexible material has guide elements (128) on its outside (126), which elements ease insertion and positioning of the body (122) in curved hollow shafts.

11. Medical cleaning device of any one of claims 1 through 10, **characterized in that** at least one opening (110) is present on the body in the region of the connection (28), by means of which opening the cleaning liquid flows into the reduced inner lumen (112).

12. Medical cleaning device of any one of claims 1 through 11, **characterized in that** the support is designed as a coupling.

## Revendications

1. Dispositif de nettoyage médical, destiné à nettoyer des surfaces internes (44, 125, 135, 152) de tiges creuses (34, 130, 134, 144) d'instruments médicaux (32), comprenant un corps fermé (12, 92, 102, 122, 136, 146) pouvant être enfiché périodiquement, en vue d'une opération de nettoyage, dans la tige creuse (34, 130, 134, 144) de l'instrument médical (32) devant être nettoyé, un diamètre extérieur (54, 84) dudit corps étant plus petit qu'un diamètre intérieur (52) de la tige creuse (34, 130, 134, 144), de telle sorte qu'il subsiste une cavité intérieure (58, 112, 138) réduite par rapport à un espace intérieur de ladite tige creuse (34, 130, 134, 144), ledit corps (12, 92, 102, 122, 136, 146) étant pourvu d'un raccord (28) par l'intermédiaire duquel, à partir d'une extrémité (16), un fluide de nettoyage peut être introduit dans l'intégralité de la cavité intérieure réduite (58, 112, 138), depuis une extrémité (36) de la tige creuse (34, 130, 134, 144), lorsque ledit corps (12, 92, 102, 122, 136, 146) est à l'état enfiché dans ladite tige creuse (34, 130, 134, 144) ; et un système de retenue (20, 106) par l'intermédiaire duquel ledit corps (12, 92, 102, 122, 136, 146) peut être périodiquement retenu sur ledit instrument médical (32) au cours d'une opération de nettoyage.

2. Dispositif de nettoyage médical selon la revendication 1, **caractérisé par le fait que** la géométrie du corps (12, 92, 102, 122, 136, 146) est choisie de façon telle qu'une section transversale de la cavité intérieure réduite (58, 112, 138) soit inférieure ou égale à la section transversale (50) du raccord (28).

3. Dispositif de nettoyage médical selon la revendication 1 ou 2, **caractérisé par le fait que** le corps est muni, à sa face extérieure, d'éléments (76, 78, 96, 128) influençant l'écoulement.

4. Dispositif de nettoyage médical selon la revendication 3, **caractérisé par le fait que** les éléments, influençant l'écoulement, sont notamment sélectionnés parmi des protubérances (96), de préférence des protubérances du type ailettes ou du type anneaux, des protubérances hélicoïdales ; des renfoncements (76), en particulier des renfoncements hélicoïdaux ou du type anneaux ; des aspérités.

5. Dispositif de nettoyage médical selon l'une des revendications 1 à 4, **caractérisé par le fait que** le corps (12, 92, 102, 122, 136, 146) revêt la forme d'une baguette.

6. Dispositif de nettoyage médical selon l'une des revendications 1 à 5, **caractérisé par le fait que** la longueur du corps peut être modifiée par aboutage de segments de corps.

7. Dispositif de nettoyage médical selon l'une des revendications 1 à 6, **caractérisé par le fait que** la géométrie de la section transversale du corps (12, 92, 102, 122, 136) correspond à la géométrie de la section transversale de la tige creuse (34, 130, 134).

8. Dispositif de nettoyage médical selon l'une des revendications 1 à 6, **caractérisé par le fait qu'**en présence de tiges creuses (144) à formes irrégulières comportant, de ce fait, des zones (150) de surfaces internes difficiles à nettoyer, le corps (146) dudit dispositif de nettoyage est pourvu, au moins dans la région de ces zones (150) de surfaces internes, d'éléments (128) influençant l'écoulement et provoquant un écoulement turbulent du fluide de nettoyage.

9. Dispositif de nettoyage médical selon l'une des revendications 1 à 8, **caractérisé par le fait que** le corps (122) est constitué d'un matériau doué de souplesse, de telle sorte qu'il puisse être enfiché également dans des tiges creuses courbes (130).

10. Dispositif de nettoyage médical selon la revendication 9, **caractérisé par le fait que** le corps (122) en matériau souple est muni, à sa face extérieure (126), d'éléments directeurs (128) qui facilitent une insertion et un positionnement dudit corps (122) dans des tiges creuses courbes (130).

11. Dispositif de nettoyage médical selon l'une des revendications 1 à 10, **caractérisé par** la présence sur le corps, dans la région du raccord (28), d'au moins un orifice (110) par l'intermédiaire duquel le fluide de nettoyage afflue dans la cavité intérieure réduite (112).

12. Dispositif de nettoyage médical selon l'une des revendications 1 à 11, **caractérisé par le fait que** le système de retenue est réalisé sous la forme d'un accouplement.
